# EUROPEAN PATENT SPECIFICATION

(11) **EP 3 266 481 B1**
(45) Date of publication and mention of the grant of the patent: **07.09.2022**
(21) Application number: 17180202.8
(22) Date of filing: 07.07.2017
(51) Int. Cl.: A61M 5/168

(54) **SYSTEM AND METHOD FOR IDENTIFYING A FILL VOLUME OF A FLUID CHAMBER**
SYSTEM UND VERFAHREN ZUR IDENTIFIZIERUNG EINES FÜLLVOLUMENS IN EINER FLÜSSIGKEITSKAMMER
SYSTÈME ET PROCÉDÉ POUR IDENTIFIER UN VOLUME DE REMPLISSAGE D'UNE CHAMBRE DE FLUIDE

(30) Priority: 08.07.2016 US 201662359911 P
(43) Date of publication of application: 10.01.2018
(73) Proprietor: Bayer Healthcare LLC, Whippany, NJ 07981 (US)
(72) Inventor: SCHRIVER, Ralph, Tarentum, PA 15084 (US); UBER, Arthur, Pittsburgh, 15208 (US)
(74) Representative: BIP Patents

(56) References cited:
- WO-A1-2009/025996
- WO-A1-2014/160307
- US-A1- 2008 086 087

## Description

### BACKGROUND OF THE DISCLOSURE

### Field of the Disclosure

The present disclosure relates generally to a system for determining the amount of fluid remaining in a fluid chamber to be injected into a patient by a syringe and/or other fluid injector, a method for the same, and a display for the same. In other aspects, the present disclosure relates to systems and methods for identifying the various features and properties of the fluid within the syringe or other fluid chamber.

### Description of Related Art

In many medical diagnostic and therapeutic procedures, a medical practitioner, such as a physician, nurse, or medical technician, injects a patient with one or more medical fluids. In recent years, a number of injector-actuated syringes and fluid injectors for pressurized injection of fluids, such as a contrast imaging agent solution (often referred to as "contrast"), a flushing agent, such as saline, and other medical fluids have been developed for use in procedures such as angiography, computed tomography ("CT"), ultrasound, magnetic resonance imaging ("MRI"), nuclear medicine, positron emission tomography ("PET"), and other imaging procedures. In general, these fluid injectors are designed to deliver a preset amount of fluid at a preset flow rate.

In some injection procedures, the medical practitioner places a catheter or a needle connected to tubing, or other fluid delivery connection into a vein or artery of the patient. The catheter or the tubing is connected to either a manual or a powered automatic fluid injection mechanism. Automatic fluid injection mechanisms typically include a connector to a fluid injector having, for example, at least one powered linear piston and/or a peristaltic pump. The syringe may include a source of contrast and/or a source of flushing fluid. The medical practitioner enters settings into an electronic control system of the fluid injector for a fixed volume of contrast and/or saline, a fixed rate of injection for each, and specific times for injections of each of the one or more fluids.

The injected contrast and/or saline are delivered to a patient's vasculature through the catheter or needle inserted into the patient's body, such as the patient's arm or groin area. A dose of contrast is referred to as a bolus. Once the bolus of contrast is delivered to the desired site, that area is imaged using a conventional imaging technique, such as angiography imaging or scanning, CT, ultrasound, MRI, PET, and/or other imaging procedures. The presence of the contrast becomes clearly visible against the background of the surrounding tissue.

Conventional injector design includes a controller, including soft buttons and readouts, located on the face of the injector assembly which requires the user or technician to enter injection data, such as the volume of fluids to be injected into a patient, and monitor the injection while remaining within arms-length of the injector assembly. An injection procedure cannot properly be completed when the supply of a fluid to be injected becomes exhausted before the procedure is completed. Thus, it is important for a user to track the volume of fluid that is in fluid communication with the injector in order to ensure that the supply of a fluid to be injected remains available during the injection procedure. One known system and method for doing so involves a user of a fluid injector manually entering a volume of a fluid or fluids into the controller or another computer. The processor of the controller or other computer includes software that tracks the amount of fluid or fluids injected into a patient, and a computer processor calculates the volume of fluid or fluids remaining over time based on an initial volume entered by the user. The calculated remaining volumes of contrast, saline, and/or other fluid to be injected may be displayed on a graphical user interface ("GUI") for the benefit of the user. Another known system is for the injector to know the volume of fluid in its syringes based on the plunger position in the syringe, assuming that the user has properly filled and confirmed that the syringes are full of the proper fluid.

However, the above systems and methods may be vulnerable to user error. For example, a user may inadvertently enter an incorrect value of the volume of fluid or fluids in fluid communication with the injector. This may occur, for example, due to mislabeling of a fluid container, or by simply inadvertently pressing the wrong button.

In addition, since most medical fluids used with power injectors are clear or only slightly tinted, and it may be difficult for a technician to quickly and easily distinguish when fluid is present in a translucent syringe or other fluid chamber. Accordingly, a need exists for a system used with a fluid injection device that is capable of differentiating between air and different types of fluid. In addition, automated systems that can determine various properties of the fluid, for example by analyzing properties and/or changes of the interaction between electromagnetic radiation with the contents of the fluid chamber, and communicating those properties to the user, for example via a display screen, are also desirable.

Moreover, it is a concern that user error in entering the correct volume of fluid contained in a fluid chamber may result in a lack of synchronization between the calculated volume of fluid in a fluid chamber, and an actual volume. This lack of synchronization may result in a power injector system failing to complete an injection procedure, as the injector system is unable to confirm that adequate medical fluid exists to complete an injection protocol. This is of particular concern for power injectors which include peristaltic pumps or other direct infusion injector systems, and may result in unnecessary diagnostic scans and repeat scans, which in turn leads to an unnecessary waste of fluid and/or wasted radiation dose to the patient.

From WO 2009/025996 A1 medical fluid injector systems are known that detect the contents and/or volume of such contents within a syringe of the system. For example, an RF signal from a first antenna of a medical fluid injector may be transmitted through a syringe associated with the medical fluid injector. At least some of the transmitted RF signal may be received by a second antenna of the medical fluid injector. An amount of the RF signal received by the second antenna may be measured to provide information regarding the contents and/or volume of such contents within the syringe. However, WO 2009/025996 A1 does not disclose determining the position of the meniscus at the liquid-gas interface

Thus, a need exists for a system and method to accurately track the volume of fluid available to be injected into a patient during an injection procedure.

### SUMMARY OF DISCLOSURE

Accordingly, it is an object of the present disclosure to provide a system and method for monitoring the remaining volume of fluid or fluids in communication with a fluid injector that overcomes various deficiencies in the prior art. To accomplish these objectives, the present invention provides a fluid injection system according to claim 1 and the claims dependent thereon, as well as a method according to claim 7 and the claims dependent thereon.

Non-limiting examples of fluid injection or delivery systems consistent with the disclosure herein are found in U.S. Patent No. 7,094,216; U.S. Provisional Application Serial No. 62/363,668; International Application Publication Nos. WO 2016/112163; WO 2016/172467; WO 2016/191485; WO 2017/040152; and International Application No. PCT/US2017/036941. Additional examples of suitable fluid injectors are disclosed in the following references: U.S. Patent No. 7,556,619; U.S. Patent No. 8,337,456; U.S. Patent No. 8,147,464; and U.S. Patent Application Publication No. 2008/0086087.

While multiple examples of systems and methods for identifying a fill volume of a fluid chamber are shown in the accompanying Figures and described herein in detail, other examples will be apparent to, and readily made by, those skilled in the art without departing from the scope of the disclosure. For example, it is to be understood that this disclosure contemplates that, to the extent possible, one or more features of any example can be combined with one or more features of any other example. Accordingly, the foregoing description is intended to be illustrative rather than restrictive.

### BRIEF DESCRIPTION OF THE DRAWINGS

For the purpose of facilitating understanding of this disclosure, the accompanying drawings and description illustrate certain embodiments, from which the various discussed structures, construction, method of operation, and many advantages provided by this disclosure may be understood and appreciated.
**FIG. 1** is a top perspective view of an embodiment of a system including a fluid injector and at least one syringe;
**FIG. 2** is a perspective view of a fluid injection system in accordance with the present disclosure;
**FIG. 3** is a perspective view of a drip chamber in accordance with an aspect of the present disclosure and adapted for use in the fluid injection system of **FIG. 2**;
**FIG. 4** is a perspective view of a fluid level sensing mechanism;
**FIG. 5** is an exploded perspective view of a fluid level sensing mechanism of **FIG. 4**;
**FIG. 6** is a transverse cross sectional view of a fluid level sensing mechanism of **FIG.** 4;
**FIG. 7** is a schematic view of a fluid chamber and sensor according to the present disclosure;
**FIG. 8A** is a schematic view of a fluid chamber and sensor arrangement according to the present disclosure;
**FIG. 8B** is a schematic view of another fluid chamber and sensor arrangement according to the present disclosure;
**FIG. 9A** is a graphical representation of signal strength as a function of a liquid-gas interface position according to an example according to the present disclosure comprising three sensors;
**FIG. 9B** is a graphical representation of signal strength as a function of a liquid-gas interface position according to an example according to the present disclosure comprising three sensors with a more gradual sensor response than in **FIG. 9A**;
**FIG. 9C** is a graphical representation of signal strength as a function of a liquid-gas interface position according to an example according to the present disclosure comprising one sensor;
**FIG. 9D** is a graphical representation of signal strength as a function of a liquid-gas interface position according to an example according to the present disclosure comprising one sensor with a more gradual sensor response than in **FIG. 9C**;
**FIG. 9E** is a graphical representation of signal strength as a function of a liquid-gas interface position according to another example according to the present disclosure;
**FIG. 9F** is a graphical representation of signal strength as a function of a liquid-gas interface position according to another example according to the present disclosure;
**FIG. 10** is a schematic view of a user interface for a fluid injection system according to the present disclosure;
**FIG. 11** is a schematic view of an example of a fluid delivery system diagram in accordance with the present disclosure;
**FIG. 12** is a flow chart of a method for determining the volume of fluid remaining within a syringe utilizing image processing techniques in accordance with an aspect of the present disclosure;
**FIG. 13** is a schematic view of an example of a fluid chamber superimposed over a vertical position scale; and
**FIG. 14** is a flow chart of a method for determining the volume of fluid remaining within a syringe utilizing image processing techniques in accordance with an aspect of the present disclosure.

### DETAILED DESCRIPTION

For purposes of the description hereinafter, spatial orientation terms, as used, shall relate to the referenced example as it is oriented in the accompanying drawing figures or otherwise described in the following detailed description. Unless stated otherwise, reference numbers cited in a particular figure refer only to the features shown in that Figure. However, it is to be understood that the embodiments described hereinafter may assume many alternative variations and configurations. It is also to be understood that the specific components, devices, and features illustrated in the accompanying drawings and described herein are simply exemplary and should not be considered as limiting.

For purposes of the description herein, the terms "upper", "lower", "right", "left", "vertical", "horizontal", "top", "bottom", "lateral", "longitudinal", and derivatives thereof shall relate to the disclosure as it is oriented in the drawing figures. When used in relation to a syringe, the term "proximal" refers to the portion of a syringe nearest to an injector, when a syringe is connected to an injector. The term "distal" refers to the portion of a syringe farthest away from an injector. It is to be understood, however, that the disclosure may assume alternative variations and step sequences, except where expressly specified to the contrary. It is also to be understood that the specific devices and processes illustrated in the attached drawings, and described in the following specification, are simply exemplary embodiments of the disclosure. Hence, specific dimensions and other physical characteristics related to the embodiments disclosed herein are not to be considered as limiting.

As used herein, the terms "electronic communication" and "electronically communicate" refer to the receipt or transfer of one or more signals, messages, commands, or other type of data. For one unit or device to be in communication with one or more other unit or device means that the one unit or device is able to receive data from and/or transmit data to the one or more other unit or device. An electronic communication may use a direct or indirect connection, and may be wired and/or wireless in nature. Additionally, two or more units or devices may be in electronic communication with each other even though the data transmitted may be modified, processed, routed, etc., between the first and second unit or device. For example, a first unit may be in electronic communication with a second unit even though the first unit passively receives data and does not actively transmit data to the second unit. As another example, a first unit may be in electronic communication with a second unit if an intermediary unit processes data from one unit and transmits processed data to the second unit. In non-limiting examples, an electronic communication may occur through one or more wired or wireless connections, such as, through one or more wires, through direct wireless protocols such as Bluetooth, Near Field Communication (NFC), or other radio frequency protocols, and/or through indirect wireless communication such as through a local Wi-Fi network or a secure Internet connection. Wireless communication may include, but is not limited to, any electronic communication that does not require direct wired contact between the two electronically communicating units or devices such as via a Wi-Fi network, communication via Bluetooth, NFC, other conventional wireless systems, or other non-wired electromagnetic communication systems. It will be appreciated that numerous other arrangements are possible.

It is to be understood that the term "fluid chamber" as it used in the present disclosure, is inclusive of the terms "syringe," "fluid container," and "drip chamber."

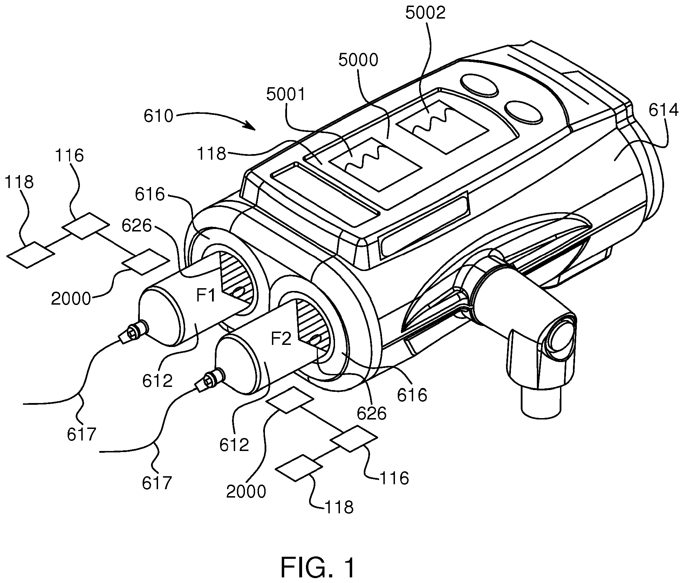

With reference to **FIG. 1**, an example of a fluid injector **610** (also referred to as "injector **610**"), such as an automated or powered fluid injector, is adapted to interface with and actuate at least one syringe **612**, each of which may be independently filled with a medical fluid **F1** and **F2**, such as contrast media, saline solution, or any desired medical fluid. The injector **610** may be used during a medical procedure to inject the medical fluid **F1,F2** into the body of a patient by driving a plunger **626** of the at least one syringe **612** with at least one piston. The injector **610** may be a multi-syringe injector, as illustrated, wherein several syringes **612** may be oriented in a side-by-side or other arrangement and include plungers **626** separately actuated by respective pistons associated with the injector **610**. In certain aspects with two syringes **612** arranged in a side-by-side relationship and filled with two different medical fluids, the injector **610** may deliver fluid from one or both of the syringes **612** either independently (single flow) or simultaneously (dual flow).

With continued reference to **FIG. 1**, the injector **610** may have a housing **614** formed from a suitable structural material, such as plastic or metal. The housing **614** may have various shapes and sizes depending on a desired application. For example, the injector **610** may be a free-standing structure configured to be placed on the floor with a stationary or movable platform. Alternatively, the injector **610** may be configured for placement on a suitable table or support frame, or hung from the ceiling. The injector **610** includes at least one syringe port **616** for receiving the at least one syringe **612** to engage respective piston elements. In some aspects, the at least one syringe **612** includes at least one syringe retaining member for retaining the syringe **612** within the syringe port **616** of the injector **610**. The at least one syringe retaining member (not shown) operatively engages a locking mechanism provided on or in the syringe port **616** of the injector **610** to facilitate loading and/or removal of the syringe **612** to and from the injector **610**, as described herein. The syringe retaining member and the locking mechanism together define a connection interface for connecting the syringe **612** to the injector **610**.

With continued reference to **FIG. 1**, at least one fluid path **617** may be fluidly connected with the at least one syringe **612** for delivering medical fluid **F** from the at least one syringe **612** to a catheter, needle, or other fluid delivery device (not shown) inserted into a patient at a vascular access site. Fluid flow from the at least one syringe **612** may be regulated by a fluid control module (not shown) comprising a processor. The fluid control module may operate various pistons, valves, and/or flow regulating structures to regulate the delivery of the medical fluid, such as saline solution and contrast, to the patient based on user-selected injection parameters, such as injection flow rate, duration, total injection volume, and/or ratio of contrast media and saline.

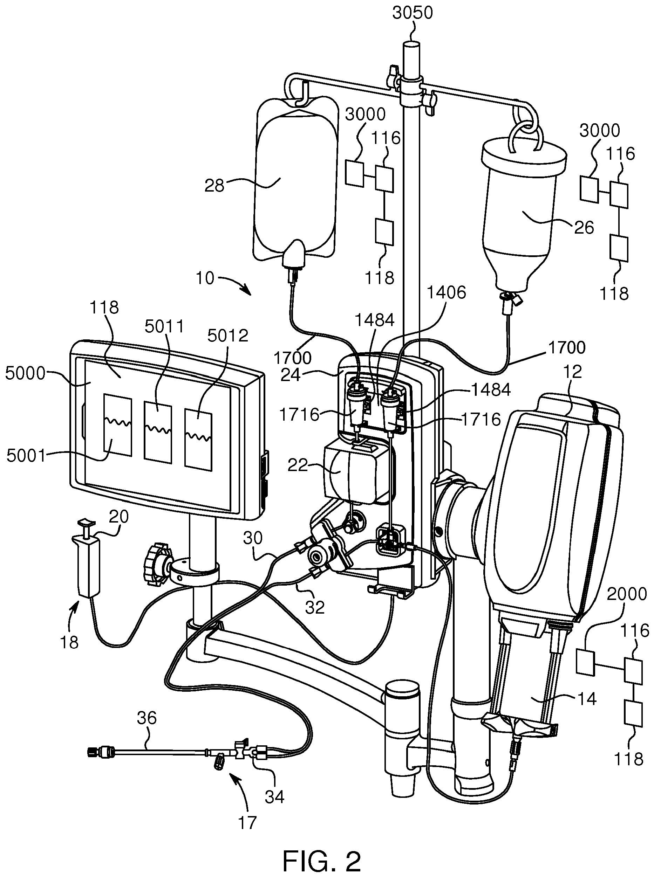

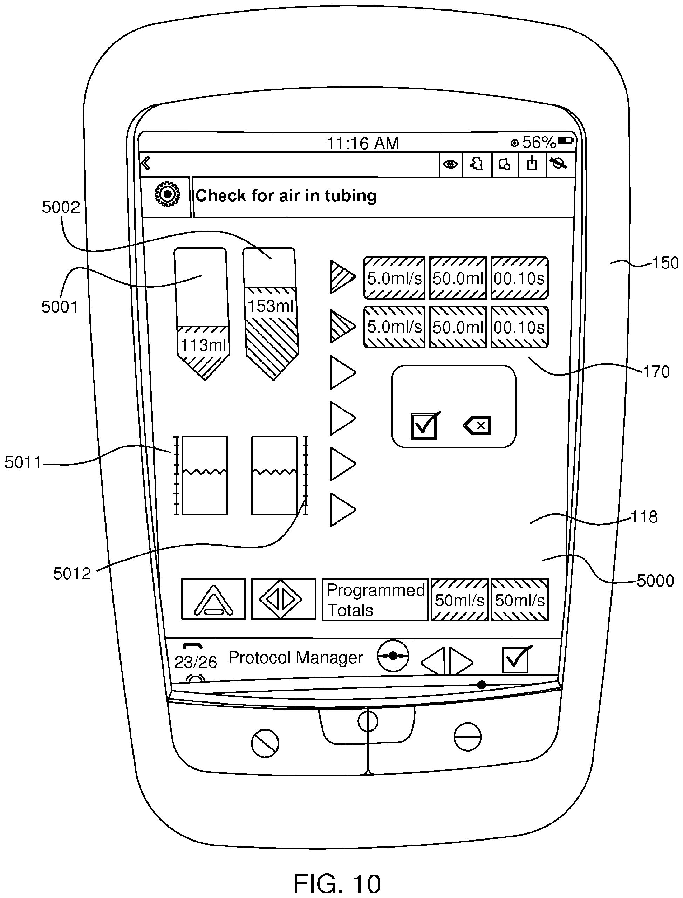

As schematically shown in **FIG. 1**, syringes **612** may be monitored by one or more sensors **2000** ("sensors **2000**"). In an embodiment, sensors **2000** may comprise optical devices that are capable of measuring the change in fill volume in syringes **612** based on measuring one or more parameters associated with the syringes **612**. In an embodiment, sensors **2000** may comprise one or more cameras. A sensor **2000** may observe the air liquid interface based upon reflection (including scatter) or transmission (including refraction) of light at that interface. The light may be supplied by a part of the injector, not shown, or may be ambient light. The sensor **2000** may observe the light that is transmitted through a syringe **612** from a pattern on the other side - for example a pattern according to the Medrad Fluidot indicator system, available from Bayer Medical Care Inc. of Indianola, Pennsylvania- or other scale or gradation patterns and thus estimate the fluid's optical index of refraction in addition to the presence and/or position of any fluid air interface, see also International Application Publication WO 2017/040154. It is also to be understood that references to sensors **2000** according to the present disclosure may include a single sensor, or an array of multiple sensors. Sensors **2000** electronically communicate by a wired mechanism, or wirelessly, with a control module, or another processor **116** comprising appropriate image recognition software that can be used to automatically identify the remaining fill volume of the syringes **612** as an injection procedure is underway, without the need of a user to enter volume data into the control unit. In other embodiments, fewer or more sensors **2000** may be included in the fluid injector **610**, as appropriate. In further non-limiting examples, syringes **612** may be equipped with a magnetic tag, magnetic tape, a bar code, RF-ID tag, a label such as a barcode or QR code, or other indicator which provides information about the mass, volume, or contents of the container, whether the contents of the container have passed their useful shelf life expiration date, or other information that is useful to the user. In an embodiment, a sensor for such a label may be situated in the at least one syringe port **616**. In an embodiment, such information may be displayed on a graphical user interface ("GUI") **5000** (see also **FIGS. 2** and **10****)** on a display **118**. In an embodiment, real-time data of the change in volume, in syringes **612**, calculated by a processor **116** may be displayed on the GUI **5000**, as may be a warning when fill volumes approach a predetermined level, or the fluids have been exhausted. Calculated fill volumes may be
shown numerically and/or graphically. Graphical representations **5001** and **5002** of fill volumes in syringes **12** are depicted on GUI **5000** in **FIG. 1**.

In an alternative embodiment, the fill volume of syringes **612** may be measured by their masses or by a correlation between the concentrations (g/mL) of a contrast agent dissolved in a volume of solvent to make up the contrast media solution. Syringes **612** may be in communication with measuring devices, not shown, that electronically communicate with a control module, or another processor comprising appropriate software to calculate the fill volume in the containers based on their masses during an injection procedure. In an embodiment, measuring devices may be included in the at least one syringe port **616**.

**FIG. 2** is a perspective view of another fluid injector **10**. The fluid injector **10** is used to deliver fluids to a patient during a medical injection procedure. For example, the fluid injector **10** may be used during an angiographic procedure to inject contrast media and common flushing agents, such as saline, into the body of a patient. An example of such a fluid injection or delivery system is disclosed in U.S. Patent No. 7,094,216.

As depicted in **FIG. 2**, fluid injector **10** generally may include a powered fluid injector **12** that is adapted to support and actuate a syringe **14** storing a first injection fluid for injection into a patient during a medical procedure, such as an angiographic procedure. The injector **12** is generally used to supply the contrast media under pressure to the fluid path set **17** and, ultimately, the patient. The injector **12** is optionally controlled by a hand controller **18** to supply the contrast media at discrete and preselected flow rates based on a physical input such as a trigger plunger **20.** The fluid injector **10** further includes a second injection fluid that may be mixed with the first injection fluid prior to being delivered to a patient or delivered directly to the patient without mixing, depending on the mode of operation of the injector **12**. The second fluid is advanced by a pumping mechanism **22** such as a peristaltic pump.

With further reference to **FIG. 2**, the powered injector **12** is operatively associated with a fluid control module **24**. The fluid control module **24** is generally adapted to support the fluid path set **17**. The fluid path set **17** is comprises one or more fluid path elements adapted to fluidly connect the syringe **14** to a container of contrast media **26** and a container of saline **28**, which is supplied to the patient via the same catheter as the contrast media **26**.

With further reference to **FIG. 2**, containers **26** and **28** of first and second injection fluids may be monitored by one or more sensors **3000** ("sensors **3000**"). In an embodiment, sensors **3000** may comprise one or more optical devices that are capable of measuring the change in fill volume in containers **26** and **28**, respectively. In an example, sensors **3000** may include one or more cameras. It is to be understood that references to "sensors" according to the present disclosure may include a single sensor, more than one sensor, a plurality of sensors, or an array of multiple sensors. Sensors **3000** electronically communicate, for example by a wired mechanism, a wireless mechanism, or a combination of wired and wireless mechanisms, with the control module **24**, or another processor **116**, comprising appropriate image recognition software that can be used to identify the remaining fill volume or amount of the containers as an injection procedure is underway, without the need of a user to enter volume or amount data into the control unit. While shown schematically in **FIG. 2** as being external to the fluid control module **24**, it is to be understood that examples of the processor **116** may physically reside in or proximate to the fluid control module **24**.

In examples, fewer or additional sensors may be included in the fluid injector **10**, as appropriate. Although schematically represented in **FIG. 2**, sensors **3000** may be positioned in the system **10** where they may measure the fluid level in containers **26** and **28.** In an example, sensors **3000** may be positioned in or on a support pole **3050.** Sensors **3000** may be configured to detect the change in level of a meniscus of a fluid in one or more of containers **26** and **28.** In an embodiment, containers **26** and **28** may include measurement markers, and sensors **3000** may be configured to detect the position of a meniscus, or a liquid-gas interface, of the fluid in containers **26** and **28** relative to the measurement markers, which provides position data on the meniscus.

In further non-limiting examples, containers **26** and **28** may be equipped with a magnetic tag, magnetic tape, a bar code, RF-ID tag, a label such as a barcode or QR code, or other indicator which provides information about the mass, volume, and/or contents of the container, lot number and/or date of manufacture and/or whether the contents of the container have passed their useful shelf life expiration date, or other information that is useful to the user. In an embodiment, such information may be read and displayed on a GUI **5000** (see also **FIG. 10**) located on a display **118**. In an embodiment, real-time data of the change in volume or amount in containers **26** and/or **28**, calculated by a processor **116** in the control module **24** or another processor **116**, may be displayed on the GUI **5000.** In specific embodiments, a warning or alarm may be triggered when fill volumes approach a predetermined level, or the fluids have been exhausted. Graphical representations **5011** and **5012** of fill volumes in containers **26** and **28** are depicted on GUI **5000** in **FIG. 2**. Non-limiting examples of graphical representations **5011** and **5012** may include real-time video images of the fill volume levels in containers **26** and/or **28**, or electronically-generated representations of fill volume levels.

In a non-limiting example, the fill volume of containers **26** and **28** may be measured by their masses. Containers **26** and **28** may be in communication with measuring devices, not shown, that electronically communicate with a processor in the control module **24**, or another processor comprising appropriate software to calculate the fill volume in the containers based on their masses, for example using a weight percent (w/w%), weight per volume percent (w/v%), or a concentration (g/mL) of solute in solvent, during an injection procedure. A computation of the mass delivered for a given volume delivered may give an indication of density of the fluid and thus information on type of contrast or whether the fluid is saline.

With further reference to **FIG. 2**, a fill volume of the syringe **14** may be monitored by a sensor **2000.** In an example, the sensor **2000** may comprise an optical device or optical devices that are capable of measuring the change in fill volume in the syringe **14**. In an example, the sensor **2000** may include one or more cameras. The sensor **2000** electronically communicates by a wired mechanism, a wireless mechanism, or a combination of wired and wireless mechanisms, with the control module **24**, or another processor **116** comprising appropriate image recognition software that can be used to identify the remaining fill volume of the syringe **14** as an injection procedure is underway, without the need of a user to enter volume or amount data into the control unit. In examples, the sensor **2000** may be stationary, or may be actuated to follow the changing fill volume level in the syringe **14**, or may be comprised of one or more optical devices, such as cameras. The sensor **2000** may include a single optical device, or multiple optical devices configured to detect the change in fill volume of the syringe **14**. An embodiment of the sensor **2000** may include an array of optical devices configured to detect the fill volume of the syringe **14**. One or more optical devices of the sensor **2000** may be included with optics, such as lenses, that give the sensor **2000** a wide field of detection, or a narrow field of detection. In an example, the sensor **2000** may be configured to detect the change in level of a plunger position or a meniscus of a fluid in the syringe **14**. In an embodiment, the syringe **14** may include measurement markers, and the sensor **2000** may be configured to detect the position of a plunger position or a meniscus of the fluid in the syringe **14** relative to the measurement markers, which provides position data on the plunger position or the meniscus. As with sensor **3000**, sensor **2000** may electronically communicate by a wired mechanism, a wireless mechanism, or a combination of wired and wireless mechanisms, with the control module **24**, or another processor **116** comprising appropriate image recognition software that can be used to identify the remaining fill volume of the syringe **14.**

In further non-limiting examples, the syringe **14** may be equipped with a magnetic tag, magnetic tape, a bar code, RF-ID tag, a label such as a barcode or QR code, or other indicator which provides information about the mass, volume, and/or contents of the container, lot number and/or date of manufacture and/or whether the contents of the container have passed their useful shelf life expiration date, or other information that is useful to the user. In an example, such information may be displayed on a GUI **5000** (see also **FIG. 10**). In an embodiment, real-time data of the change in volume or amount in the syringe **14** may be calculated by a processor in the control module **24,** or another processor, and may be displayed on the GUI **5000.** In certain embodiments, a warning or alarm may be triggered when fill volumes approach a predetermined level, or the fluids have been exhausted. Graphical representations **5001** of the fill volume in the syringe **14** are depicted on the GUI **5000** in **FIG. 2**. Non-limiting examples of graphical representations **5001** may include a real-time video image of the fluid level in the syringe **14**, or electronically-generated representations of fill volume levels.

The fluid path set **17** may be a single-use or multi-use disposable connection including a first input line **30** and a second input line **32**, a downstream Y-connector **34** joining the first and second input lines **30**, **32**, and a catheter connector conduit **36**. Aspects of the fluid path set **17** may be found in U.S. Patent No. 7,094,216, and/or U.S. Patent No. 7,556,619e.

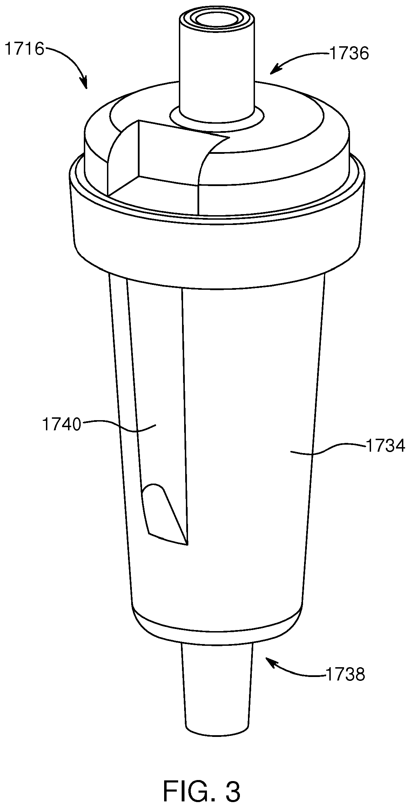

Referring briefly to **FIG. 3**, an embodiment of a drip chamber **1716** used in the fluid injector **10** of **FIG. 2** is shown in enlarged detail. The drip chamber **1716,** shown in **FIGS. 2** and **3**, generally has an elongated body **1734** with a top end **1736** and a bottom end **1738.** The body **1734** is formed with a projection **1740**, which generally extends longitudinally, laterally along the body **1734**, or in any configuration on the body **1734** of the drip chamber **1716,** and may even be in the form of a handle with an opening such as those found on plastic bottles. The projection **1740** is generally provided to interact with one or more fluid level sensing mechanisms **1406**, and may be referred to as a "back" window because the projection **1740** will generally face the fluid level sensors in the fluid level sensing mechanism **1406** when the drip chamber **1716** is associated with one or more fluid level sensing mechanisms **1406**.

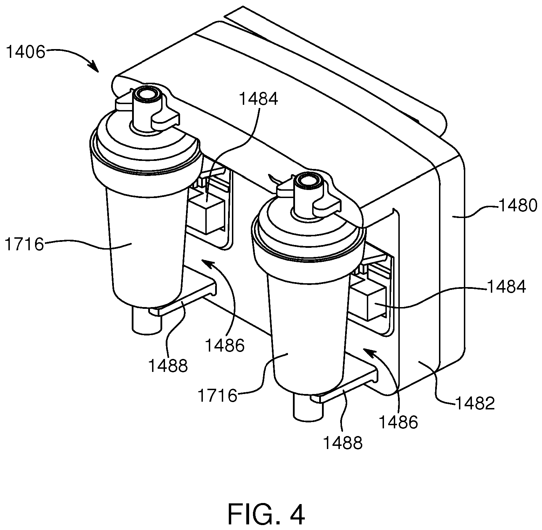

With reference to **FIGS. 3** and **4**, the body **1734** is preferably formed of a plastic material and, more particularly, a resiliently deformable medical-grade plastic material to allow in-place "priming" of the drip chamber **1716** by squeezing, when the drip chamber **1716** is associated with a fluid level sensing mechanism **1406**. The at least one fluid level sensing mechanism **1406** may be adapted to support and secure the drip chambers **1716**, or may be offset from the drip chambers **1716.** In an embodiment, the projection **1740** further permits the drip chamber **1716** to be primed in place in the fluid level sensing mechanism **1406**. The plastic material comprising the body **1734** may be substantially clear or slightly opaque, but the projection **1740** is preferably clear to allow an optical fluid sensor in the fluid level sensing mechanism **1406** to detect the fluid level in the drip chamber **1716**. In another embodiment, the material comprising the body **1734** may be comprised of a transparent or translucent material, to allow an optical fluid sensor in the fluid level sensing mechanism **1406** to detect the fluid level in the drip chamber **1716**. The projection **1740** is preferably raised from the body **1734** of the drip chamber **1716** to allow priming of the drip chamber **1716.** Generally, the body **1734** of the drip chamber **1716** is sufficiently clear to allow light transmission from lighting associated with the fluid level sensing mechanism **1406**. The body **1734** of the drip chamber **1716** will generally act as a light conduit or "light pipe" that will illuminate the fluid flow path in the medical tubing forming the output lines **1718** associated with the drip chambers **1716** connected to containers **26** and **28**.

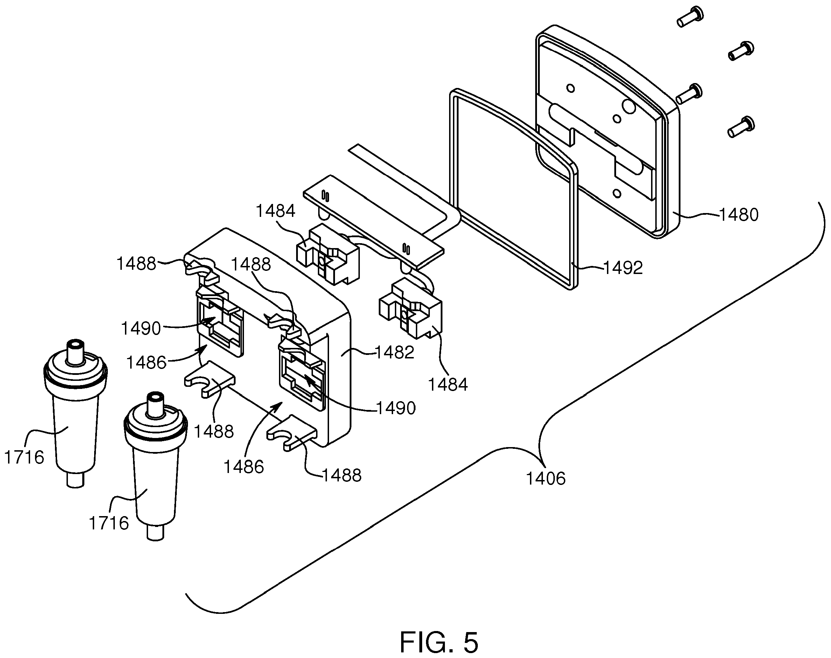

Referring to **FIGS. 4-7**, an embodiment of a fluid level sensing mechanism **1406** configured to be provided on a fluid control module is shown in greater detail. The fluid sensing mechanism **1406** generally interfaces with the drip chambers **1716** associated with the containers **26** and **28**. The fluid sensing mechanism **1406** is provided to indicate to the operator of the fluid injector that sufficient injection fluid, either primary contrast media or secondary saline, is available for an injection or flushing procedure. The fluid sensing mechanism **1406** is generally adapted to warn the operator when the fluid level in the drip chambers **1716** is below a level sufficient to conduct an injection procedure. During filling or priming, the operator may squeeze the drip chamber **1716** so that some of the air in the drip chambers **1716** moves up into the containers **26** and **28** and the fluid level, the liquid-gas interface, is at or above the desired level as measured by the fluid sensing mechanism **1406**. The fluid sensing mechanism **1406** is provided as a safety feature to ensure that air is not introduced into the fluid path set **1700** during an injection procedure or flushing procedure involving the fluid injector.

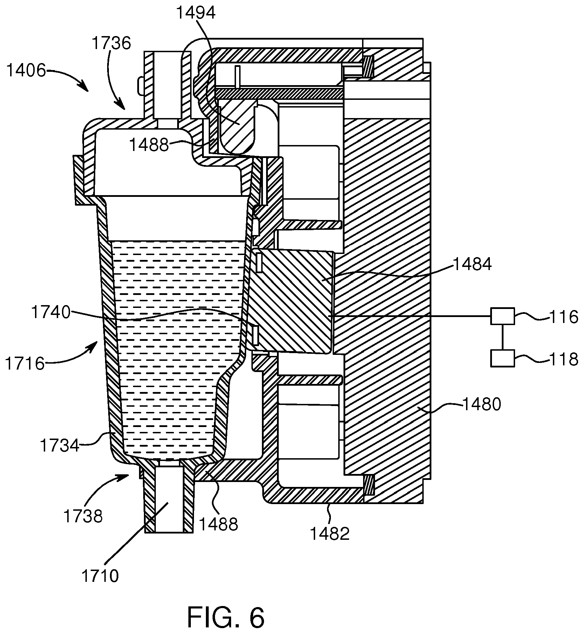

The fluid sensing mechanism **1406** generally includes a support plate **1480**, a drip chamber support **1482**, and one or more fluid level sensors **1484** (hereinafter "fluid sensors **1484**") which are adapted for association with the drip chambers **1716** connected to the fluid containers **26** and **28**. The support plate **1480** generally supports the various components of the fluid sensing mechanism **1406.** The drip chamber support **1482** is generally secured to the support plate **1480** by suitable mechanical fasteners or another suitable attachment or mounting scheme. The drip chamber support **1482** may be a unitary structure that is integrally molded of plastic material, and includes a plurality of attachment or support locations **1486** adapted to support the drip chambers **1716.** In particular, the drip chamber support **1482** may include snap mounts or positions **1488** for securing the bodies **1734** of the drip chambers **1716** in the fluid sensing mechanism **1406**, and operatively associated with the fluid sensors **1484.** The snap mounts **1488** may be adapted to engage inlet and outlet ports of the drip chambers **1716,** as shown in **FIG. 6**.

In a non-limiting example, the drip chamber support **1482** defines respective openings **1490** for receiving the fluid sensors **1484,** and associating the fluid sensors **1484** with the drip chambers **1716.** The openings **1490** are positioned to allow the fluid sensors **1484** to be operatively associated with the projection **1740** formed on the bodies **1734** of the respective drip chambers **1716.** As shown in **FIG. 6**, the fluid sensors **1484** may physically contact the projections **1740** on the drip chambers **1716,** when the drip chambers **1716** are secured in the support locations **1486** on the drip chamber support **1482.** Non-limiting examples of the fluid sensors **1484** include optical or ultrasonic sensors. It also is to be understood that references to fluid sensors **1484** according to the present disclosure may include a single sensor, or an array of multiple sensors. A suitable ultrasonic sensor for the fluid sensors **1484** is manufactured by Omron. A gasket **1492** may be provided between the drip chamber support **1482** and the support plate **1480** to prevent fluid intrusion between the drip chamber support **1482** and the support plate **1480**, which could damage the fluid sensors **1484.** Lights **1494** may be associated with the support locations **1486** to illuminate the drip chambers **1716.** The lights **1494** are further adapted to visually indicate when the fluid level in the drip chambers **1716** drops below a predetermined level during operation of the fluid injector, for example by changing modes to an intermittent mode and blinking to indicate to the operator that insufficient fluid is available for an injection procedure. The lights **1494** provide "back-lighting" for not only the drip chambers **1716** but also the medical tubing associated with the drip chambers **1716,** and light the medical tubing and drip chambers **1716** in such a manner that the medical tubing and the drip chambers **1716** form a "light pipe" that illuminates at least part if not all of the first section **1710** of the fluid path set **1700**. The back lighting allows the operator of the fluid injector to easily visually inspect the drip chambers **1716** to check the fluid level present in the drip chambers **1716**.

The fluid sensors **1484** are generally adapted to provide fluid position signals to the computer hardware/software associated with the fluid control module and/or injector to indicate the fluid levels in the drip chambers **1716.** Position of the liquid-gas interface in the drip chamber changes as a function of the amount of fluid in the associated fluid container **26** or **28**. The fluid sensors **1484** may be further adapted to initiate an alarm signal to the computer hardware/software associated with the fluid control module and/or the injector when the fluid level in the drip chambers **1716** falls below a predetermined level. The computer hardware/software associated with the fluid control module and/or the injector may be adapted to respond to the alarm signal by halting the on-going injection procedure.

As illustrated in an embodiment depicted in **FIG. 6**, the fluid sensors **1484** are tilted or angled at a slight or small angle relative to a vertical axis generally parallel to the face of the support plate **1480**. The slight angle, for example, is selected to complement the projection **1740** on the bodies **1734** of the drip chambers **1716.** The projection **1740** on the bodies of the drip chambers **1716** is preferably tapered at a small angle, such as 3 degrees. The projection **1740** on the bodies **1734** of the drip chambers **1716** is preferably tapered inward at a small angle from the top end **1736** to the bottom end **1738** on the drip chambers **1716,** as illustrated in **FIG. 6****.** The fluid sensors **1484** are positioned in the openings **1490** to complement the tapered projections **1740** on the respective drip chambers **1716,** and preferably physically contact the projections **1740** as indicated previously.

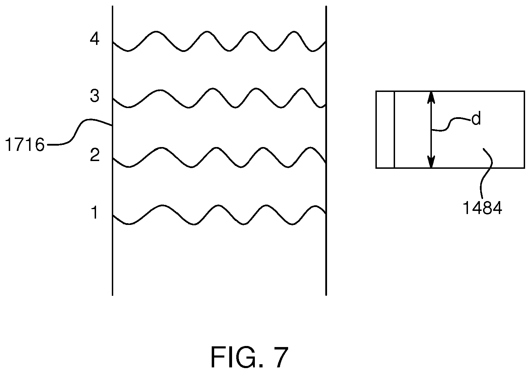

**FIG. 7** shows a schematic view of a cross section of drip chamber **1716.** While this Figure contemplates a drip chamber, it is to be understood that the description herein may apply to determining fluid levels in other fluid chambers, such as containers **26** and **28**, with sensors **3000** as well. Multiple possible fluid levels **1**, **2**, **3**, **4** are shown in the drip chamber **1716.** An exemplary single sensor **1484** of an embodiment of a fluid sensing mechanism **1406** is depicted. The sensor **1484** has a height **d**. As can be seen from the Figure, the position and height of the single sensor **1484** makes it well disposed to detect and measure the changes in the liquid-gas interface in the drip chamber **1716** when the fluid is at or above level **2**. However, as fluid drops below level **2**, the exemplary single sensor **1484** may not detect or measure the liquid-gas interface well. Similarly, when the fluid is at or above level **4**, changes in fluid level may not be detected by the fluid sensing mechanism **1406**.

Monitoring the location of a liquid-gas interface, such as the location of a meniscus, is superior to monitoring the presence or absence of fluid because it allows additional functionalities. For example, in an embodiment, by tracking the direction and/or the rate of travel of the liquid-gas interface, that information can be compared to the fill rate of the drip chamber to assess whether the fluid source has been depleted, disposable integrity, the fill pressure drop, the type of fluid being filled, and venting in the container, **26** or **28**.

By way of a non-exhaustive list of examples, if the liquid-gas interface falling rate and/or steady state or equilibrium filling position matches that expected for the injection filling rate within a pre-determined error band, this may indicate normal filling operation. If the liquid-gas interface is falling at a rate, or to a position, that is known to coincide with that of an empty supply, that may be an indication that the fluid source has been depleted. If the liquid-gas interface is falling at a rate or to a position not commensurate with the filling rate, it may indicate an obstruction with the supply venting or a clogged spike filter. If, after the filling stops, the liquid-gas interface is not rising at a rate or a level commensurate with the normal drip chamber filling rate, it may indicate that the fluid container, **26** or **28** is not properly vented. The movement and/or level of the liquid-gas interface or fluid amount may also be used as input to the injector control system or other means to adjust or affect the fill rate in a closed loop or open loop fashion. Any or all of these conditions may be displayed for an operator on a GUI **5000** on a display **118**, as seen in **FIG. 10**, or result in an audible alarm, in order to notify the operator of the condition, or a processor **116** or the fluid control module **24** automatically enabling or preventing the injector **10** from performing an action.

In an embodiment, the liquid-gas interface level may be monitored for the purpose of permitting a "smart fill" process. The injector filling operation could tie the servo control to the liquid-gas interface position and speed up or slow down the filling as needed. This would allow the operator to fill lower viscosity contrast agents and saline at a faster rate, thus optimizing workflow. This feature could not only self-compensate for high viscosity fluids, but may also manage variation in bottle sizes, venting performance, heating, disposable set variation, and other benefits as well.

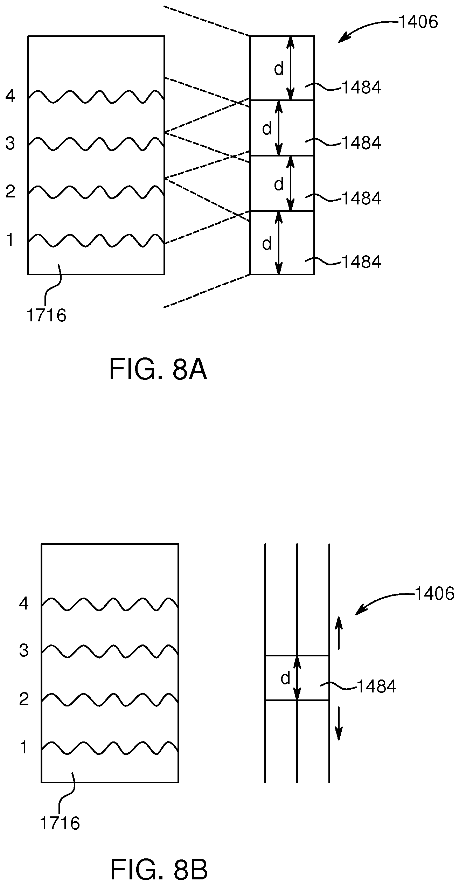

**FIG. 8A** shows a schematic view of a cross section of a drip chamber and a non-limiting example of a fluid sensing mechanism **1406.** According to the example, the fluid sensing mechanism **1406** includes an array of sensors **1484** configured to detect a liquid-gas interface at multiple levels in the drip chamber **1716.** As shown, each sensor **1484** has a height **d**; however, sensors **1484** of differing heights could be used. Four sensors **1484** are shown to make the array; however various numbers of sensors could be used. For example, a linear array of sensors or a two dimensional array such as a camera may be used. In the case of optical sensors, sensors **1484** may be outfitted with optics to give them separated, contiguous, or overlapping fields of detection. The dashed lines of **FIG. 8A** represent the fields of detection of the sensors **1484.** The sensors **1484** are in electronic communication with a processor, and software could be used to determine the level of the liquid-gas interface, and changes therein, with a higher degree of accuracy and over a greater range, than if just a single sensor **1484** were used. This could be accomplished, for example, by comparing the signal strengths and/or changes in the signal strengths of the various sensors **1484** in the array to each other, to a composite of the total signal, or to some otherwise determined level.

In an embodiment depicted in **FIG. 8B**, a single sensor **1484** may be actuated or moved to follow the liquid-gas interface based on changes in the signal strength. Position data of the sensor **1484** may be communicated to a processor, and from those data, the level of the liquid-gas interface in drip chamber **1716** may be calculated by a processor. An array of sensors **1484** may also be actuated.

In embodiments depicted in both **FIGS. 8A** and **8B**, the fluid sensing mechanism **1406** may be positioned closely to the drip chamber **1716,** or be offset a distance from it, in order to give sensors **1484** a wider field of detection. The fluid sensing mechanism **1406** may be configured to support the drip chamber **1716,** or may be situated by the drip chamber **1716** without supporting it.

It is to be understood that examples according to **FIGS.** 7 and **8A****-8B** may be applied to sensors **3000** configured to detect fill volumes in containers **26** and **28** as well.

With reference to **FIGS. 2**, 4-7, and 8A-8B, the fluid injector **10** may be configured as an image recognition system that includes at least one sensor **3000** and/or **1484,** such as an image capture device, positioned having a field of detection directed to a container **26**, **28**; a processor **116** including a controller operatively connected to the sensor **3000** and/or **1484** and configured to process the images obtained from the sensor **3000** and/or **1484** using suitable image processing software; and a display **118** operatively connected to the processor **116** for displaying the results of the image processing performed by the central processing unit. In one example, the image processing software may be the Insight Explorer software from Cognex Corporation of Natick, MA and the sensor **3000** and/or **1484** may be a DataMan 100 camera also from Cognex Corporation. In addition, the at least one sensor **3000** and/or **1484** and the processor **116** may be integrated into a single component or provided as individual components. Further, the at least one sensor **3000** and/or **1484,** the fluid injector **10,** the display **118**, and/or the processor **116** may be in wired communication or may communicate wirelessly, for example via Bluetooth, Wi-Fi, or other conventional wireless communication technology, or combination of various wired and/or wireless communication methods. Further, with reference to **FIGS. 1** and **2**, it is to be understood that the above examples of software and configuration may be used for sensors **2000** as well.

In another example, with reference to **FIGS. 2**, 4-7, and 8A-8B the sensors **3000** and/or **1484** can be an alternative type of sensor, such as an electromagnetic radiation detector, ultrasonic detector, or other suitable sensor as is known in the art, or even combinations of the various sensor types described herein. In some examples, the at least one sensor **3000** and/or **1484** is a digital camera that can be configured to obtain a digital image of a liquid-gas interface in a container **26**, **28**, and/or a drip chamber **1716,** respectively. In other examples, the at least one sensor **3000** and/or **1484** can be an infrared radiation detector, ultraviolet light detector, ultrasound imaging device, or any other suitable sensor for identifying electromagnetic radiation emitted from an electromagnetic radiation source, not shown. With reference to **FIGS. 1** and **2**, it is to be understood that the above configuration may be used for sensors **2000** as well.

As will be appreciated by one of ordinary skill in the art, the at least one sensor **3000** and/or **1484** or detector can be adapted specifically for identifying a wavelength of electromagnetic radiation or light associated with an electromagnetic radiation source, not shown, and the illuminated identification pattern produced therewith. For example, the at least one sensor **3000** and/or **1484** can include various filters or tuned or attenuated optical elements for identifying only radiation within an expected wavelength (e.g., electromagnetic radiation within a wavelength emitted by the electromagnetic radiation source). Additionally, the containers **26**, **28**, the projection **1740** of the drip chamber **1716,** or the drip chamber **1716** themselves can be used as filters by altering the material properties (e.g., color, molecular alignment, pigment additive, polarized surface) to filter light of a given wavelength to achieve an optimized visualization by the user. Alternatively, image processing techniques can be used to remove portions of obtained images outside of the expected wavelength, thereby reducing an influence of ambient light and increasing sensitivity for the illuminated identification pattern. With reference to **FIGS. 1** and **2**, it is to be understood that the above configuration may be used for sensors **2000** as well.

**FIG. 10** shows a GUI **5000** of a display **118** on a user interface **150** in accordance with a non-limiting example of the present disclosure, showing various injection parameters **170** for an injection procedure, displayed on a touch screen. As described herein and/or known to those skilled in the art, the various parameters **170** may be monitored, changed, or inputted by the user before, during, or after an injection procedure, for example by touching the appropriate field on the touch screen and entering the appropriate data using an electronic keypad that appears on the touch screen. In certain examples, some or all of the parameters may be saved and uploaded to a patient records database either wirelessly or by wired connection from the user interface or another computer, for example a hospital information system or network. In non-limiting configurations, various parameters may be saved and uploaded automatically and/or in response to a user command. Graphical representations **5001** and **5002** of liquid-gas interfaces and/or plunger positions in syringes **612** or **14**, and/or graphical representations **5011** and **5012** of liquid-gas interfaces in drip chambers **1716** or containers **26, 28** may be shown on the GUI **5000** in **FIG. 10****,** and may be updated in real time, or at pre-determined time intervals, based on data provided by the sensors **1484**, **2000**, **3000.** The GUI **5000** may show graphical representations of single or multiple syringes, and/or containers, and/or drip chambers **1716**, depending on the needs of the user. In examples, a user may be able to choose which liquid-gas interface levels to display on the GUI **5000**. Non-limiting examples of graphical representations **5001**, **5002**, **5011**, and **5012** may include real-time video images of liquid-gas interfaces in drip chambers **1716** and/or containers **26, 28** as well as plunger position and/or or gas presence in syringes **14** or **612**, or electronically-generated representations of liquid-gas interface levels, for example such that the user may check or confirm visually. As shown in **FIG. 10**, graphical representations of liquid-gas interface levels in drip chambers may be accompanied by numerical values as well. Alternatively, the GUI **5000** may show only numerical values of liquid-gas interface levels. Additional data, such as the rate of change liquid-gas interface levels, also may be displayed on the GUI **5000.** Alternatively, the GUI **5000** may blink or give some other visual indication of any alarm or other states of the system, for example those discussed herein in relation to the activation of the light associated with the drip chambers **1716**. Optionally the displayed color of the fluid, the fluid shape outline, or some other on screen indication may change color based upon the amount of fluid, for example green when full, yellow when in an intermediate range and red when in the lowest range.

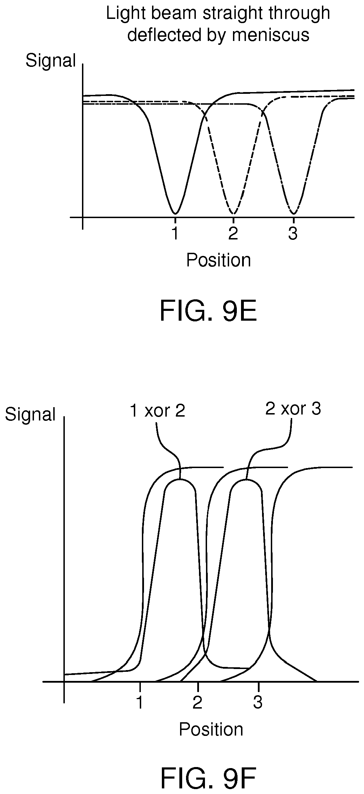

With reference to **FIGS. 1**, **2**, and **6**, sensors **2000**, **3000**, and **1484** may comprise one or more electro-magnetic sensors, photosensitive detectors, ultrasonic sensors, or any sensor known in the art that may be used to detect the position of the liquid-gas interface and/or plunger position, or combinations of different sensor types. One non-limiting example of a detection mechanism including a sensor according to the present disclosure includes a beam of electromagnetic energy, such as visible, ultraviolet, or infrared light, passing through the fluid in a fluid chamber to a photosensitive sensor. In such a configuration, the beam is transmitted whether there is air or liquid in its path, but the intensity of the beam received by the sensor will be affected if the liquid-gas interface is in the beam's path. **FIG. 9E** depicts a graphical representation of signal strength as a function of the position of the liquid-gas interface using a configuration of three such sensors. However, it should be understood that some examples may include more or fewer sensors.

With continuing reference to **FIG. 9E**, the dips in signal strength on the graph correspond to situations wherein the liquid-gas interface passes within the field of detection of a sensor. In such a configuration, the apparatus according to the present disclosure may allow a user and/or the system to determine the position of the liquid-gas interface at various critical points in the filling process, depending on the positioning of the sensors according to this example.

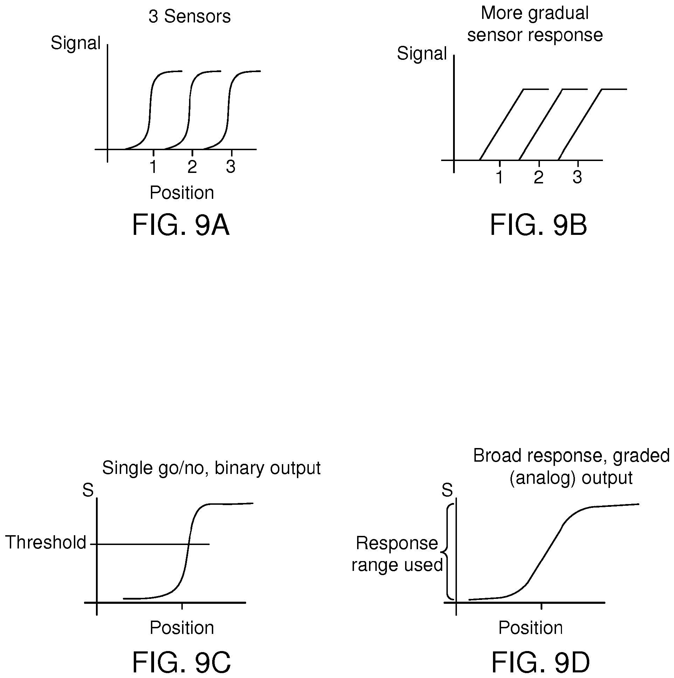

In another example, the sensors may differentiate between the presence and absence of liquid in its field of detection. A non-limiting example of such a sensor is an ultrasonic sensor. **FIGS. 9A-9D** show graphical depictions of signal strength as a function of position of a liquid-gas interface in various configurations of sensors using sensors that differentiate between the presence and absence of liquid in their fields of detection. In such a configuration, as the liquid-gas interface passes through the field of detection, the signal changes from low to high or high to low.

**FIG. 9C** depicts signal strength for a configuration using one such sensor with a narrow field of detection. As the liquid-gas interface passes through the field of detection of the sensor, the signal strength changes relatively rapidly for a change in interface position. Sensors according to this example allow a user to determine whether the liquid-gas interface is above or below the field of detection of the sensor. A sensor with a response as shown in **FIG. 9C** which responds fully over a relatively small range compared to the full range of fluid in the fluid chamber may be considered to be a point or a zero dimensional or "0D" sensor. In a non-limiting embodiment, the sensor may be actuated and, for example, oscillate between an upper and lower position, for example by adjustments in fluid flow rate, allowing the liquid-gas interface to pass repeatedly through the field of detection of the sensor. This may be referred to as "dithering" in the art. Dithering returns data on the position of the liquid-gas interface because the signal strength of the sensor will change as the sensor's field of detection crosses the liquid-gas interface. Dithering effectively makes a zero dimensional sensor a one dimensional sensor. The sensors **2000**, **3000**, and/or **1484** according to this example may be configured to dither between set positions that correspond to desired fluid levels within the container. If data indicate that the liquid-gas interface is lower or higher than a desired level for too long or moves with an inappropriate rate, an error value may be returned, and/or an alarm could be sounded alerting the user of the error and optionally providing a proposed solution to the error alert. A processor **116** may stop, change or enable an action according to such data. This is especially important in the case that the data returned by the sensor indicates that the liquid-gas interface is lower than the desired level, because it may indicate that an insufficient amount of liquid is in the fluid chamber.

**FIG 9D** depicts a graphical representation of signal strength of a single sensor in a configuration using a sensor with a wider field of detection, or a more gradual response than that used in **FIG. 9C**. In an embodiment according to this configuration, as the liquid-gas interface travels through the field of detection of the sensor, there is a more gradual relationship between the position of the liquid-gas interface and the signal output of the sensor. In an example according to this configuration, data returned by the sensor may be generally proportional or univariately related to the position of the liquid-gas interface over a range of the sensor's field of detection, and the sensor's reading may be used to assess the position of the liquid-gas interface. In an example, the sensor's reading may also be used to determine a range of dithering, and/or to trigger error states, alarms, and/or the allowance or cessation of an action by the injection system. A single sensor with an extended field of detection may be sufficient to provide a one dimensional measurement that is used to determine the liquid gas interface position in the fluid chamber or container. A scale or other mass (or weight) determining sensor may also be configured to have a response as shown in **FIG. 9D**. A time of flight ultrasound or sound transducer measuring the sound transmission and reflection time from the bottom or top of the fluid container respectively to the fluid interface may be configured to have a response as shown in the figure as well. Other single sensor fluid level systems know in the art may be configured to have a response such as shown in **FIG. 9D**. Sensors such as listed here, a one dimensional optical array, a two dimensional optical array (for example, a camera) as well as sensors known to those skilled in the art may provide the 1D or one dimensional measurement that serves as input to determining the fluid fill of the fluid chamber.

It may be preferable to configure the fluid sensors **2000**, **3000**, **1484** to detect liquid-gas interface at a plurality of levels. **FIG. 9A** shows a graph depicting signal strength in a configuration of three sensors **1484** as a function of the position of the liquid-gas interface in a fluid chamber. It is to be understood, however, that such a configuration may be used with respect to sensors **2000**, **3000.** **FIG. 9A** shows a graphical depiction of signal strength for a configuration using three sensors that differentiate between the presence and absence of liquid, with narrow fields of detection. However, it should be understood that more or fewer sensors may be used. In the configuration according to **FIG. 9A**, information returned by the sensors indicates whether the liquid-gas interface is above or below the levels of a container within the fields of detection of the sensors. A position of the liquid-gas interface may be determined by comparing sensor signals. This comparison may be made by using an exclusive-or gate, which also may be referred to as an "xor" or "exor" gate in the art. As depicted in **FIG. 9F**, the output of the xor gate is high when one, and only one, of the two signal outputs that it compares is high. Thus, when the output of sensor one is high, but sensor two is not, then the signal retuned from the xor gate comparing sensors one and two would be high. If the signal detected by sensors one and two of this configuration both are low or high, then the xor gate comparing sensors one and two is low. Similar results are returned for the xor gate comparing sensors two and three. Thus, the use of one or more xor gates provides data on the position of the liquid-gas interface.

Thus, a configuration of two sensors with narrow fields of detection allows for the detection of whether the liquid-gas interface is below both, between the two (which may correspond to a controlled, or optimum, range of fluid levels in the container), or above both. In a further embodiment, a third, middle sensor may be provided between an upper and a lower sensor. This middle sensor may be actuated to allow it to move slightly, to provide more precise data on the position of the liquid-gas interface in the space between the fields of detection of the upper and lower sensors.

**FIG. 9B** depicts a graphical representation of signal strength of a sensor for a configuration using a sensor with a wider field of detection, or more gradual transition than that used in **FIG. 9A**. The fields of detection or regions of transition may be separated, contiguous, or overlapping.

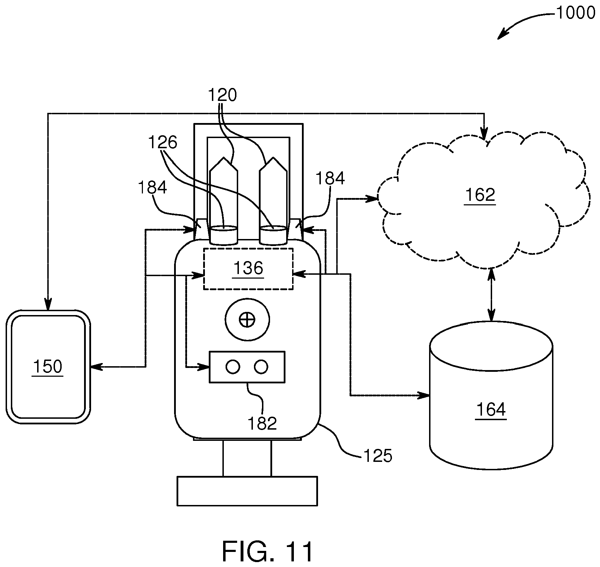

**FIG. 11** illustrates a system **1000** for controlling a fluid delivery in accordance with a non-limiting example of the present disclosure. In the non-limiting example shown, a fluid control device **136** comprising at least one processor may be formed as part of or connected to a housing **125** and configured or programmed to control an injection and/or fluid delivery in a syringe **120** engaged in a syringe port **126**.

In the non-limiting example shown, the fluid control device **136** is in wired and/or wireless communication, as described herein, with a user interface **150** such as a removable touch screen tablet computer or other computer interface, a network **162**, a patient records database **164**, a plurality of syringe identification sensors **184**, and a secondary controller **182**. In further non-limiting examples, the user interface **150** may be further configured to be in wired or wireless communication with a medical scanner, an injection protocol database, or other device or system and to allow a user to download, upload, display, and/or manipulate data from the medical scanner, injection protocol database, and/or other systems related to the medical injection procedure.

In the non-limiting example of **FIG. 11**, the fluid control device **136** may be provided with container identification sensors **184** associated with each fluid interface, such as a syringe port **126** provided in **FIG. 11**, or containers **26** and **28** and/or drip chambers **1716** of **FIG. 2**, and in communication with the processor of the fluid control device **136** as well as with the user interface **150.** Each container identification sensor **184** may comprise a sensor such as a camera, a radio frequency receiver, an optical label reader, a magnetic sensor, an optical sensor, a mechanical sensor, or any combination thereof, which is configured to detect identifying information about a syringe **120** or other fluid chamber engaged within its corresponding syringe port and communicate this information to the processor of the fluid control device **136** and/or user interface **150**. Container identification sensors **184** may receive fill volume data from syringes optically, for example, by tracking the changes in plunger position and/or meniscus level of a fluid in a syringe, or by change in mass. Container identification sensors **184** may transmit this data to the fluid control device **136** or another processor comprising appropriate image recognition software used to identify a change in fill volume without input from a user. The fill volume, and any changes therein, may be displayed on the GUI **5000** of the user interface **150.**

In an example according to the present disclosure, data on the liquid-gas interface may be synchronized with the fluid control module **24**, the processor **116**, or other processor.

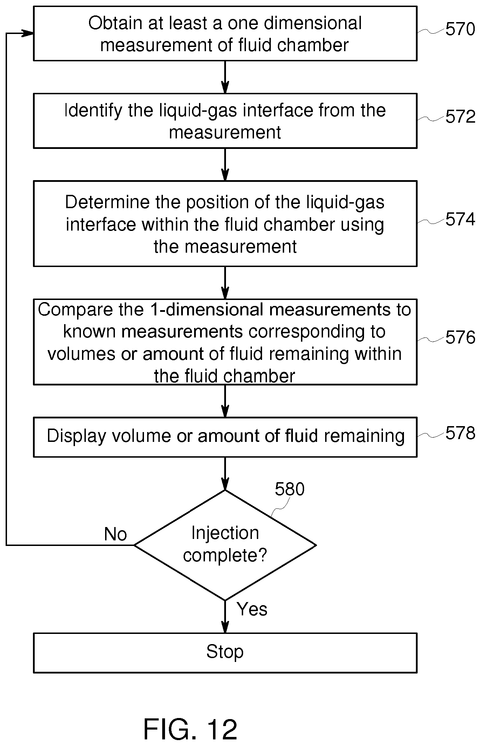

In one example, the fluid injector **10** of **FIG. 2** may be arranged such that the at least one sensor **3000**, **1484** can take a measurement or measurements of the liquid-gas interface in a container **26**, **28**, and/or drip chamber **1716**, respectively. Based on these measurements, the volume, weight, or concentration of contrast or saline remaining within the containers **26**, **28** can be determined. Specifically, with reference to **FIG. 12****,** an at least one dimensional measurement of the container **26**, **28**, is obtained by sensors **3000** at step **570.** Then, at step **572**, the measurement processing software identifies the liquid-gas interface in the measurement as described herein. Next, at step **574**, the measurement processing software determines the position of the liquid-gas interface within the container **26, 28** by determining the change in location of the liquid-gas interface relative to a reference point. In certain embodiments, steps **572** and **574** may be used for example during an optical measurement and may be optional but not necessary for other means of measuring, for example measurements of weight of the fluid using a scale. Once the position of the liquid-gas interface within the container **26**, **28** has been determined, this position can be compared to known positions corresponding to a volume or amount of fluid remaining within the containers **26**, **28** at step **576**. The processor **116** then sends a signal to display the volume or amount of fluid remaining to the display **118** at step **578**. The volume or amount remaining may be displayed as a numerical value, or a graphical representation of the container **26, 28** may be displayed that illustrates the real-time volume or amount remaining within the container. Data are taken at a sufficient rate and the display of volume or amount remaining is updated at a sufficient rate until the injection procedure is complete as determined at step **580.** In an example according to the present disclosure, imaging or other software on the processor **116**, or another processor operatively connected to the fluid injector **10**, may automatically stop the injection procedure at step **580**. It will be understood that this method may be applied to determining the fluid volume or amount in a drip chamber **1716** with the sensor **1484.** It will be further understood that in an example according to the present disclosure, the method described herein may be performed with image data taken of the liquid-gas interface by an optical device such as a camera.

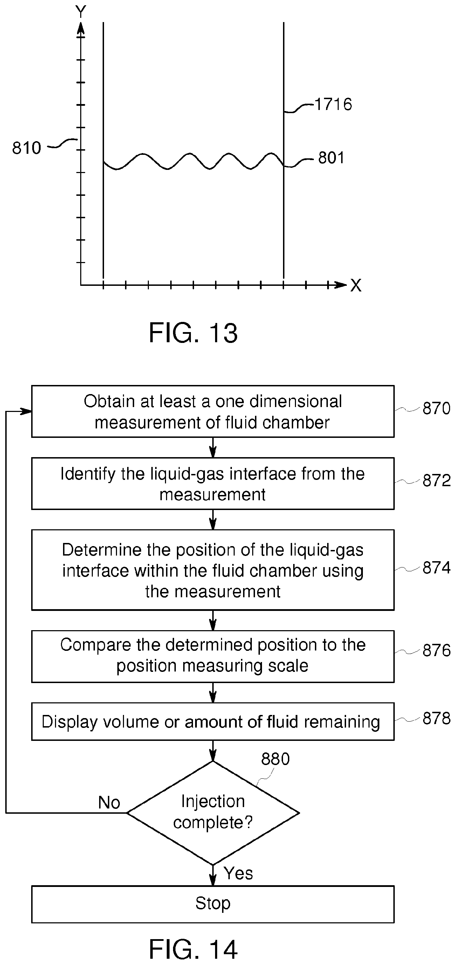

**FIG. 13** shows a schematic representation of a cross section of a drip chamber **1716** viewed from the perspective of a sensor **1484,** with a position measuring scale **801**. While this Figure contemplates a drip chamber, it is to be understood that the description herein may apply to determining fluid levels in other fluid chambers, such as bulk containers **26** and **28**, with sensors **3000** as well. In an example according to the present disclosure, a position measuring scale **810** may be physically located on the drip chamber **1716,** or otherwise physically in view of the sensor **1484.** In examples, the measuring scale **810** may be electronically superimposed in a graphical representation **5011**, **5012** by imaging software or other software on the processor **116** (see **FIG. 10**). The sensor **1484** is positioned or otherwise calibrated so that the detected position of the liquid-gas interface **801** corresponds to an actual vertical level of the liquid-gas interface in the liquid chamber. When the liquid-gas interface reaches a predetermined level, as detected by the sensor **1484,** an operator may be notified. As shown in **FIG. 13**, position the measuring scale **810** may be behind, a part of, or imposed over the drip chamber **1716,** or other fluid chamber. In an example according to the present disclosure, imaging or other software on the processor **116**, or another processor operatively connected to the fluid injector **10**, may automatically continue, or stop the injection procedure based on the detected position of the liquid-gas interface **801** with respect to the measuring scale **810.**

With reference to **FIG. 14**, an at least one dimensional measurement of the bulk fluid container **26, 28**, is obtained by sensors **3000** at step 870. Then, at step **872**, the measurement processing software identifies the liquid-gas interface in the measurement as discussed herein. Next, at step **874**, the measurement processing software determines the position of the liquid-gas interface within the bulk fluid container **26, 28** by determining the position of the liquid-gas interface relative to a level on the measuring scale **810**. Once the position of the liquid-gas interface within the container **26**, **28** has been determined, this position can be compared to known positions corresponding to a volume or amount of fluid remaining within the containers **26**, **28** at step **876**. The processor **116** then sends a signal to display the volume or amount remaining to the display **118** at step **878.** The volume or amount remaining may be displayed as a numerical value or a graphical representation of the container **26**, **28** may be displayed that illustrates the real-time volume or amount remaining within the container. Data may be taken at a sufficient rate and the display of volume remaining is updated at a sufficient rate until the injection procedure is complete as determined at step **880**. In an example according to the present disclosure, imaging or other software on the processor **116**, or another processor operatively connected to the fluid injector **10**, may automatically stop the injection procedure at step **880** based on the detected position of the liquid-gas interface **801**. It will be understood that this method may be applied to determining the fluid volume or amount in another fluid chamber, such as a drip chamber **1716** with a sensor **1484**. It will be further understood that in an example according to the present disclosure, the method described herein may be performed with image data taken of the liquid-gas interface by an optical device such as a camera.

Although the disclosure has been described in detail for the purpose of illustration based on what is currently considered to be the most practical and preferred embodiments, it is to be understood that such detail is solely for that purpose and that the disclosure is not limited to the disclosed embodiments.

## Claims

1. A fluid injection system comprising:
a fluid injector (610, 10);
at least one fluid chamber (612, 26, 28, 1716) configured to contain fluid, the at least one fluid chamber (612, 26, 28, 1716) in fluid communication with the fluid injector (610, 10);
one or more electromagnetic radiation or ultrasonic sensors (2000, 3000, 1484) positioned relative to the at least one fluid chamber (612, 26, 28, 1716) to detect a position of a meniscus at a liquid-gas interface (801) of the fluid contained in the at least one fluid chamber (612, 26, 28, 1716) by dithering a field of detection of the one or more, electromagnetic radiation or ultrasonic sensors; and
at least one processor (116) in communication with the one or more electromagnetic radiation or ultrasonic sensors (2000, 3000, 1484) and the fluid injector (610, 10), the at least one processor (116) configured to:
determine the position of the meniscus at the liquid-gas interface of the fluid in the at least one fluid chamber (612, 26, 28, 1716);
calculate the volume of fluid contained in the at least one fluid chamber (612, 26, 28, 1716) based on the position of the meniscus at the liquid-gas interface of the fluid in the at least one fluid chamber (612, 26, 28, 1716), and at least one of:
i) display on a display (118) in communication with the at least one processor (116) the volume of the fluid contained in the at least one fluid chamber (612, 26, 28, 1716);
ii) enable the fluid injector (610, 10) to perform an injector filling operation or an injection procedure;
iii) inform a user of an insufficient volume and allow the user to install a fluid chamber (612, 26, 28, 1716) having a sufficient volume;
iv) inform the user of the insufficient volume and allow the user to continue with a system-adjusted volume or a user-adjusted volume; and
v) disable the fluid injector (610, 10) from performing the injector filling operation or an injection procedure.

2. The fluid injection system of claim 1,
wherein the fluid injector (610, 10) comprises a pump (22) and is in fluid communication with a fluid path set (17, 1700);
wherein the at least one fluid chamber (612, 26, 28, 1716) further comprises at least one drip chamber (1716) in fluid communication with the fluid injector (610, 10) and at least one of a contrast media container (26) and a saline container (28); and
wherein at least one of the one or more sensors (2000, 3000, 1484) is positioned relative to the at least one drip chamber (1716) to detect the position of the meniscus at the liquid-gas interface (801) of the fluid contained in the at least one drip chamber (1716).

3. The fluid injection system of claim 1 or 2, wherein the one or more electromagnetic radiation sensors (2000, 3000, 1484) comprise an optical sensor or an ultrasonic sensor.

4. The fluid injection system of any one of claims 1 to 3, wherein the display (118) comprises a graphical user interface (5000), and wherein the position of the meniscus at the liquid-gas interface (801) of the fluid in the at least one fluid chamber (612, 26, 28, 1716) is displayed on the display (118) graphical user interface (5000).

5. The fluid-injection system of any one of claims 1 to 4, wherein the one or more electromagnetic radiation or ultrasonic sensors (2000, 3000, 1484) comprise an array of electromagnetic radiation or ultrasonic sensors (2000, 3000, 1484).

6. The fluid-injection system of any one of claims 1 to 5, further comprising an actuator configured to actuate the one or more electromagnetic radiation or ultrasonic sensors (2000, 3000, 1484), wherein the one or more electromagnetic radiation or ultrasonic sensors (2000, 3000, 1484) are actuated to move in response to the position of the meniscus at the liquid-gas interface (801) of the fluid in the at least one fluid chamber (612, 26, 28, 1716).

7. A method for determining the volume of fluid in at least one fluid chamber (612, 26, 28, 1716) of a fluid injection system according to claim 1, the method comprising the steps of:
positioning one or more electromagnetic radiation or ultrasonic sensors (2000, 3000, 1484) relative to the at least one fluid chamber (612, 26, 28, 1716), wherein the one or more electromagnetic radiation or ultrasonic sensors (2000, 3000, 1484) are in communication with at least one processor (116) in communication with the fluid injector (610, 10);
dithering a field of detection of the one or more electromagnetic radiation or ultrasonic sensors (2000, 3000, 1484) to detect a position of a meniscus at a liquid-gas interface (801) of the fluid contained in the at least one fluid chamber (612, 26, 28, 1716);
taking position data of the meniscus at the liquid-gas interface (801) of the fluid contained in the at least one fluid chamber (612, 26, 28, 1716) with at least one of the one or more electromagnetic radiation or ultrasonic sensors (2000, 3000, 1484);
determining the position of the meniscus at the liquid-gas interface (801) of the fluid in the at least one fluid chamber (612, 26, 28, 1716) from the position data;
calculating the volume of fluid contained in the at least one fluid chamber (612, 26, 28, 1716) based on the position of the meniscus at the liquid-gas interface (801) of the fluid in the at least one fluid chamber (612, 26, 28, 1716); and at least one of:
i) displaying on a display (118) in communication with the at least one processor (116) the volume of the fluid contained in the at least one fluid chamber (612, 26, 28, 1716);
ii) enabling the fluid injector (610, 10) to perform an injector filling operation or an injection procedure;
iii) informing a user of an insufficient volume and allowing the user to install a fluid chamber (612, 26, 28, 1716) having a sufficient volume;
iv) informing the user of the insufficient volume and allowing the user to continue with a system-adjusted volume or a user-adjusted volume; and
v) disabling the fluid injector (610, 10) from performing the injector filling operation or an injection procedure.

8. The method according to claim 7, wherein the steps of enabling the fluid injector (610, 10) to perform the function, and disabling the fluid injector (610, 10) from performing the injector filling operation or an injection procedure, are automatically completed by the at least one processor (116).

9. The method according to claim 7 or 8, wherein the one or more sensors (2000, 3000, 1484) comprise a one dimensional optical device.

10. The method according to any one of claims 7 to 9, further comprising the step of determining the volume of fluid contained in the at least one fluid chamber (612, 26, 28, 1716) by comparing the position data with known positions corresponding to known volumes of fluid.

11. The method according to any one of claims 7 to 10, further comprising the steps of:
positioning a position measuring scale (810) within a field of detection of the one or more sensors (2000, 3000, 1484);
comparing the position data with the positioning measuring scale (810); and
determining the volume of fluid contained in the at least one fluid chamber (612, 26, 28, 1716) based on a relative position of the meniscus at the liquid-gas interface (801) and a value indicated by the measuring scale (810).

12. The method according to any one of claims 7 to 11, wherein the fluid injector (610, 10) comprises a pump (22), and the fluid injector (610, 10) in fluid communication with a fluid path set (17, 1700);
wherein the at least one fluid chamber (612, 26, 28, 1716) further comprises at least one drip chamber (1716) in fluid communication with the fluid injector (610, 10) and at least one of a contrast media container (26) and a saline container (28); and
wherein at least one of the one or more electromagnetic radiation or ultrasonic sensors (2000, 3000, 1484) is positioned relative to the at least one drip chamber (1716) to detect the position of the meniscus at the liquid-gas interface (801) of the fluid contained in the at least one drip chamber (1716).

13. The method according to any one of claims 7 to 12, wherein the display (118) comprises a graphical user interface (5000), and wherein the position of the meniscus at the liquid-gas interface (801) of the fluid in the at least one fluid chamber (612, 26, 28, 1716) is displayed on the graphical user interface (5000).

14. The method according to any one of claims 7 to 13, wherein the fluid injector (610, 10) comprises an actuator configured to actuate at least one of the one or more electromagnetic radiation or ultrasonic sensors (2000, 3000, 1484), and further comprising the step of:
actuating the one or more electromagnetic radiation or ultrasonic sensors (2000, 3000, 1484) to move in response to the position of the meniscus at the liquid-gas interface (801) of the fluid in the at least one fluid chamber (612, 26, 28, 1716).

## Patentansprüche

1. Fluidinjektionssystem, umfassend:
einen Fluidinjektor (610, 10);
mindestens eine Fluidkammer (612, 26, 28, 1716), die zum Enthalten von Fluid ausgelegt ist, wobei die mindestens eine Fluidkammer (612, 26, 28, 1716) mit dem Fluidinjektor (610, 10) in Fluidverbindung steht;
einen oder mehrere elektromagnetische Strahlungssensoren oder Ultraschallsensoren (2000, 3000, 1484), die in Bezug auf die mindestens eine Fluidkammer (612, 26, 28, 1716) so positioniert sind, dass sie eine Position eines Meniskus an einer Flüssigkeits-Gas-Grenzfläche (801) des in der mindestens einen Fluidkammer (612, 26, 28, 1716) enthaltenen Fluids durch Dithern eines Detektionsfeldes des einen oder der mehreren elektromagnetischen Strahlungssensoren oder Ultraschallsensoren detektieren; and
mindestens einen Prozessor (116) in Kommunikation mit dem einen oder den mehreren elektromagnetischen Strahlungssensoren oder Ultraschallsensoren (2000, 3000, 1484) und dem Fluidinjektor (610, 10), wobei der mindestens eine Prozessor (116) ausgelegt ist zum:
Bestimmen der Position des Meniskus an der Flüssigkeits-Gas-Grenzfläche des Fluids in der mindestens einen Fluidkammer (612, 26, 28, 1716);
Berechnen des Volumens des in der mindestens einen Fluidkammer (612, 26, 28, 1716) enthaltenen Fluids basierend auf der Position des Meniskus an der Flüssigkeits-Gas-Grenzfläche des Fluids in der mindestens einen Fluidkammer (612, 26, 28, 1716), und mindestens einem von:
i) Anzeigen des Volumens des in der mindestens einen Fluidkammer (612, 26, 28, 1716) enthaltenen Fluids auf einer Anzeige (118) in Kommunikation mit dem mindestens einen Prozessor (116);
ii) Aktivieren des Fluidinjektors (610, 10) zum Durchführen eines Injektor-Füllvorgangs oder einer Injektionsprozedur;
iii) Informieren eines Benutzers von einem unzureichenden Volumen und Ermöglichen, dass der Benutzer eine Fluidkammer (612, 26, 28, 1716) mit einem ausreichenden Volumen installiert;
iv) Informieren des Benutzer vom unzureichenden Volumen und Ermöglichen, dass der Benutzer mit einem durch das System angepassten Volumen oder einem vom Benutzer angepassten Volumen fortfährt; und
v) Deaktivieren des Fluidinjektors (610, 10) zum Unterbinden des Injektor-Füllvorgangs oder einer Injektionsprozedur.

2. Fluidinjektionssystem nach Anspruch 1,
wobei der Fluidinjektor (610, 10) eine Pumpe (22) umfasst und mit einem Fluidpfadsatz (17, 1700) in Fluidverbindung steht;
wobei die mindestens eine Fluidkammer (612, 26, 28, 1716) ferner mindestens eine Tropfkammer (1716) in Fluidverbindung mit dem Fluidinjektor (610, 10) und mindestens einen von einem Kontrastmittelbehälter (26) und einem Kochsalzlösungsbehälter (28) umfasst; und
wobei mindestens einer des einen oder der mehreren Sensoren (2000, 3000, 1484) in Bezug auf die mindestens eine Tropfkammer (1716) so positioniert ist, dass er die Position des Meniskus an der Flüssigkeits-Gas-Grenzfläche (801) des in der mindestens einen Tropfkammer (1716) enthaltenen Fluids detektiert.

3. Fluidinjektionssystem nach Anspruch 1 oder 2, wobei der eine oder die mehreren elektromagnetischen Strahlungssensoren (2000, 3000, 1484) einen optischen Sensor oder einen Ultraschallsensor umfassen.

4. Fluidinjektionssystem nach einem der Ansprüche 1 bis 3, wobei die Anzeige (118) eine grafische Benutzeroberfläche (5000) umfasst, und wobei die Position des Meniskus an der Flüssigkeits-Gas-Grenzfläche (801) des Fluids in der mindestens einen Fluidkammer (612, 26, 28, 1716) auf der grafischen Benutzerfläche (5000) der Anzeige (118) angezeigt wird.

5. Fluidinjektionssystem nach einem der Ansprüche 1 bis 4, wobei der eine oder die mehreren elektromagnetischen Strahlungssensoren oder Ultraschallsensoren (2000, 3000, 1484) ein Array von elektromagnetischen Strahlungssensoren oder Ultraschallsensoren (2000, 3000, 1484) umfassen.

6. Fluidinjektionssystem nach einem der Ansprüche 1 bis 5, ferner umfassend einen Aktor, der zum Betätigen des einen oder der mehreren elektromagnetischen Strahlungssensoren oder Ultraschallsensoren (2000, 3000, 1484) ausgelegt ist, wobei der eine oder die mehreren elektromagnetischen Strahlungssensoren oder Ultraschallsensoren (2000, 3000, 1484) betätigt werden, um sich in Reaktion auf die Position des Meniskus an der Flüssigkeits-Gas-Grenzfläche (801) des Fluids in der mindestens einen Fluidkammer (612, 26, 28, 1716) zu bewegen.

7. Verfahren zur Bestimmung des Volumens von Fluid in mindestens einer Fluidkammer (612, 26, 28, 1716) eines Fluidinjektionssystems nach Anspruch 1, wobei das Verfahren die folgenden Schritte umfasst:
Positionieren eines oder mehrerer elektromagnetischer Strahlungssensoren oder Ultraschallsensoren (2000, 3000, 1484) in Bezug auf die mindestens eine Fluidkammer (612, 26, 28, 1716), wobei der eine oder die mehreren elektromagnetischen Strahlungssensoren oder Ultraschallsensoren (2000, 3000, 1484) mit einem Prozessor (116) in Kommunikation sind, der mit dem Fluidinjektor (610, 10) in Kommunikation ist;
Dithern eines Detektionsfeldes des einen oder der mehreren elektromagnetischen Strahlungssensoren oder Ultraschallsensoren (2000, 3000, 1484), um eine Position eines Meniskus an einer Flüssigkeits-Gas-Grenzfläche (801) des in der mindestens einen Fluidkammer (612, 26, 28, 1716) enthaltenen Fluids zu detektieren;
Aufnehmen von Positionsdaten des Meniskus an der Flüssigkeits-Gas-Grenzfläche (801) des in der mindestens einen Fluidkammer (612, 26, 28, 1716) enthaltenen Fluids mit mindestens einem des einen oder der mehreren elektromagnetischen Strahlungssensoren oder Ultraschallsensoren (2000, 3000, 1484);
Bestimmen der Position des Meniskus an der Flüssigkeits-Gas-Grenzfläche (801) des Fluids in der mindestens einen Fluidkammer (612, 26, 28, 1716) aus den Positionsdaten;
Berechnen des Volumens des in der mindestens einen Fluidkammer (612, 26, 28, 1716) enthaltenen Fluids basierend auf der Position des Meniskus an der Flüssigkeits-Gas-Grenzfläche (801) des Fluids in der mindestens einen Fluidkammer (612, 26, 28, 1716); und mindestens eines von Folgendem:
i) Anzeigen des Volumens des in der mindestens einen Fluidkammer (612, 26, 28, 1716) enthaltenen Fluids auf einer Anzeige (118) in Kommunikation mit dem mindestens einen Prozessor (116);
ii) Aktivieren des Fluidinjektors (610, 10) zum Durchführen eines Injektor-Füllvorgangs oder einer Injektionsprozedur;
iii) Informieren eines Benutzers von einem unzureichenden Volumen und Ermöglichen, dass der Benutzer eine Fluidkammer (612, 26, 28, 1716) mit einem ausreichenden Volumen installiert;
iv) Informieren des Benutzer vom unzureichenden Volumen und Ermöglichen, dass der Benutzer mit einem durch das System angepassten Volumen oder einem vom Benutzer angepassten Volumen fortfährt; und
v) Deaktivieren des Fluidinjektors (610, 10) zum Unterbinden des Injektor-Füllvorgangs oder einer Injektionsprozedur.

8. Verfahren nach Anspruch 7, wobei die Schritte des Aktivierens des Fluidinjektors (610, 10) zum Ausführen der Funktion und Deaktivieren des Fluidinjektors (610, 10) zum Unterbinden des Injektor-Füllvorgangs und einer Injektionsprozedur durch den mindestens einen Prozessor (116) automatisch ausgeführt werden.

9. Verfahren nach Anspruch 7 oder 8, wobei der eine oder die mehreren Sensoren (2000, 3000, 1484) eine eindimensionale optische Vorrichtung umfassen.

10. Verfahren nach einem der Ansprüche 7 bis 9, ferner umfassend den Schritt des Bestimmens des Volumens des in der mindestens einen Fluidkammer (612, 26, 28, 1716) enthaltenen Fluids durch Vergleichen der Positionsdaten mit bekannten Positionen, die bekannten Volumina des Fluids entsprechen.

11. Verfahren nach einem der Ansprüche 7 bis 10, ferner umfassend die folgende Schritte:
Positionieren einer Positionsmessskala (810) innerhalb eines Detektionsfeldes des einen oder der mehreren Sensoren (2000, 3000, 1484);
Vergleichen der Positionsdaten mit der Positionsmessskala (810); und
Bestimmen des Volumens des in der mindestens einen Fluidkammer (612, 26, 28, 1716) enthaltenen Fluids basierend auf einer relativen Position des Meniskus an der Flüssigkeits-Gas-Grenzfläche (801) und einem durch die Messskala (810) angegebenen Wert.

12. Verfahren nach einem der Ansprüche 7 bis 11, wobei der Fluidinjektor (610, 10) eine Pumpe umfasst (22), und der Fluidinjektor (610, 10) mit einem Fluidpfadsatz (17, 1700) in Fluidverbindung steht;
wobei die mindestens eine Fluidkammer (612, 26, 28, 1716) ferner mindestens eine Tropfkammer (1716) in Fluidverbindung mit dem Fluidinjektor (610, 10) und mindestens einen von einem Kontrastmittelbehälter (26) und einem Kochsalzlösungsbehälter (28) umfasst; und
wobei mindestens einer des einen oder der mehreren elektromagnetischen Strahlungssensoren oder Ultraschallsensoren (2000, 3000, 1484) in Bezug auf die mindestens eine Tropfkammer (1716) so positioniert wird, dass er die Position des Meniskus an der Flüssigkeits-Gas-Grenzfläche (801) des in der mindestens einen Tropfkammer (1716) enthaltenen Fluids detektiert.

13. Verfahren nach einem der Ansprüche 7 bis 12, wobei die Anzeige (118) eine grafische Benutzeroberfläche (5000) umfasst, und wobei die Position des Meniskus an der Flüssigkeits-Gas-Grenzfläche (801) des Fluids in der mindestens einen Fluidkammer (612, 26, 28, 1716) auf der grafischen Benutzerfläche (5000) angezeigt wird.

14. Verfahren nach einem der Ansprüche 7 bis 13, wobei der Fluidinjektor (610, 10) einen Aktor umfasst, der zum Betätigen mindestens eines des einen oder der mehreren elektromagnetischen Strahlungssensoren oder Ultraschallsensoren (2000, 3000, 1484) ausgelegt ist, und ferner umfassend den folgenden Schritt:
Betätigen des einen oder der mehreren elektromagnetischen Strahlungssensoren oder Ultraschallsensoren (2000, 3000, 1484), damit sie sich in Reaktion auf die Position eines Meniskus an der Flüssigkeits-Gas-Grenzfläche (801) des Fluids in der mindestens einen Fluidkammer (612, 26, 28, 1716) bewegen.

## Revendications

1. Système d'injection de fluide comprenant :
un injecteur de fluide (610, 10) ;
au moins une chambre de fluide (612, 26, 28, 1716) configurée pour contenir un fluide, l'au moins une chambre de fluide (612, 26, 28, 1716) étant en communication fluidique avec l'injecteur de fluide (610, 10),
un ou plusieurs capteurs de rayonnement électromagnétique ou d'ultrasons (2000, 3000, 1484) positionnés par rapport à l'au moins une chambre de fluide (612, 26, 28, 1716) pour détecter une position d'un ménisque à une interface liquide-gaz (801) du fluide contenu dans l'au moins une chambre de fluide (612, 26, 28, 1716) en faisant osciller un champ de détection du ou des capteurs de rayonnement électromagnétique ou d'ultrasons ; et
au moins un processeur (116) en communication avec le ou les capteurs de rayonnement électromagnétique ou d'ultrasons (2000, 3000, 1484) et l'injecteur de fluide (610, 10), l'au moins un processeur (116) étant configuré pour :
déterminer la position du ménisque à l'interface liquide-gaz du fluide dans l'au moins une chambre de fluide (612, 26, 28, 1716) ;
calculer le volume de fluide contenu dans l'au moins une chambre de fluide (612, 26, 28, 1716) sur la base de la position du ménisque à l'interface liquide-gaz du fluide dans l'au moins une chambre de fluide (612, 26, 28, 1716), et au moins l'une des actions suivantes :
i) afficher sur un dispositif d'affichage (118) en communication avec l'au moins un processeur (116) le volume du fluide contenu dans l'au moins une chambre de fluide (612, 26, 28, 1716) ;
ii) permettre à l'injecteur de fluide (610, 10) d'effectuer une opération de remplissage de l'injecteur ou une procédure d'injection ;
iii) informer un utilisateur d'un volume insuffisant et permettre à l'utilisateur d'installer une chambre de fluide (612, 26, 28, 1716) ayant un volume suffisant ;
iv) informer l'utilisateur du volume insuffisant et permettre à l'utilisateur de continuer avec un volume ajusté par le système ou un volume ajusté par l'utilisateur ; et
v) empêcher l'injecteur de fluide (610, 10) d'effectuer l'opération de remplissage de l'injecteur ou une procédure d'injection.

2. Système d'injection de fluide selon la revendication 1,
l'injecteur de fluide (610, 10) comprenant une pompe (22) et étant en communication fluidique avec un ensemble de chemins de fluide (17, 1700) ;
l'au moins une chambre de fluide (612, 26, 28, 1716) comprenant en outre au moins une chambre de goutte-à-goutte (1716) en communication fluidique avec l'injecteur de fluide (610, 10) et au moins l'un d'un récipient de produit de contraste (26) et d'un récipient de solution saline (28) ; et
au moins un du ou des capteurs (2000, 3000, 1484) étant positionné par rapport à l'au moins une chambre de goutte-à-goutte (1716) pour détecter la position du ménisque à l'interface liquide-gaz (801) du fluide contenu dans l'au moins une chambre de goutte-à-goutte (1716).

3. Système d'injection de fluide selon la revendication 1 ou 2, le ou les capteurs de rayonnement électromagnétique (2000, 3000, 1484) comprenant un capteur optique ou un capteur à ultrasons.

4. Système d'injection de fluide selon l'une quelconque des revendications 1 à 3, le dispositif d'affichage (118) comprenant une interface utilisateur graphique (5000), et la position du ménisque à l'interface liquide-gaz (801) du fluide dans l'au moins une chambre de fluide (612, 26, 28, 1716) étant affichée sur l'interface utilisateur graphique (5000) du dispositif d'affichage (118).

5. Système d'injection de fluide selon l'une quelconque des revendications 1 à 4, le ou les capteurs de rayonnement électromagnétique ou d'ultrasons (2000, 3000, 1484) comprenant un réseau de capteurs de rayonnement électromagnétique ou d'ultrasons (2000, 3000, 1484).

6. Système d'injection de fluide selon l'une quelconque des revendications 1 à 5, comprenant en outre un actionneur configuré pour actionner le ou les capteurs de rayonnement électromagnétique ou d'ultrasons (2000, 3000, 1484), le ou les capteurs de rayonnement électromagnétique ou d'ultrasons (2000, 3000, 1484) étant actionnés pour se déplacer en réponse à la position du ménisque à l'interface liquide-gaz (801) du fluide dans l'au moins une chambre de fluide (612, 26, 28, 1716).

7. Procédé pour déterminer le volume de fluide dans au moins une chambre de fluide (612, 26, 28, 1716) d'un système d'injection de fluide selon la revendication 1, le procédé comprenant les étapes de :
positionnement d'un ou plusieurs capteurs de rayonnement électromagnétique ou d'ultrasons (2000, 3000, 1484) par rapport à l'au moins une chambre de fluide (612, 26, 28, 1716), le ou les capteurs de rayonnement électromagnétique ou d'ultrasons (2000, 3000, 1484) étant en communication avec au moins un processeur (116) en communication avec l'injecteur de fluide (610, 10) ;
oscillation d'un champ de détection du ou des capteurs de rayonnement électromagnétique ou d'ultrasons (2000, 3000, 1484) pour détecter une position d'un ménisque à une interface liquide-gaz (801) du fluide contenu dans l'au moins une chambre de fluide (612, 26, 28, 1716) ;
prise des données de position du ménisque à l'interface liquide-gaz (801) du fluide contenu dans l'au moins une chambre de fluide (612, 26, 28, 1716) avec au moins un du ou des capteurs de rayonnement électromagnétique ou d'ultrasons (2000, 3000, 1484),
détermination de la position du ménisque à l'interface liquide-gaz (801) du fluide dans l'au moins une chambre de fluide (612, 26, 28, 1716) à partir des données de position ;
calcul du volume de fluide contenu dans l'au moins une chambre de fluide (612, 26, 28, 1716) sur la base de la position du ménisque à l'interface liquide-gaz (801) du fluide dans l'au moins une chambre de fluide (612, 26, 28, 1716), et au moins l'une des actions suivantes :
i) afficher sur un dispositif d'affichage (118) en communication avec l'au moins un processeur (116) le volume du fluide contenu dans l'au moins une chambre de fluide (612, 26, 28, 1716) ;
ii) permettre à l'injecteur de fluide (610, 10) d'effectuer une opération de remplissage de l'injecteur ou une procédure d'injection ;
iii) informer un utilisateur d'un volume insuffisant et permettre à l'utilisateur d'installer une chambre de fluide (612, 26, 28, 1716) ayant un volume suffisant ;
iv) informer l'utilisateur du volume insuffisant et permettre à l'utilisateur de continuer avec un volume ajusté par le système ou un volume ajusté par l'utilisateur ; et
v) empêcher l'injecteur de fluide (610, 10) d'effectuer l'opération de remplissage de l'injecteur ou une procédure d'injection.

8. Procédé selon la revendication 7, les étapes permettant à l'injecteur de fluide (610, 10) de réaliser la fonction, et empêchant l'injecteur de fluide (610, 10) de réaliser l'opération de remplissage de l'injecteur ou une procédure d'injection, étant automatiquement réalisées par l'au moins un processeur (116).

9. Procédé selon la revendication 7 ou 8, le ou les capteurs (2000, 3000, 1484) comprenant un dispositif optique unidimensionnel.

10. Procédé selon l'une quelconque des revendications 7 à 9, comprenant en outre l'étape de détermination du volume de fluide contenu dans l'au moins une chambre de fluide (612, 26, 28, 1716) en comparant les données de position avec des positions connues correspondant à des volumes de fluide connus.

11. Procédé selon l'une quelconque des revendications 7 à 10, comprenant en outre les étapes de :
positionnement d'une échelle de mesure de positionnement (810) dans un champ de détection du ou des capteurs (2000, 3000, 1484) ;
comparaison des données de position avec l'échelle de mesure de position (810) ; et
détermination du volume de fluide contenu dans l'au moins une chambre de fluide (612, 26, 28, 1716) sur la base d'une position relative du ménisque à l'interface liquide-gaz (801) et d'une valeur indiquée par l'échelle de mesure (810).

12. Procédé selon l'une quelconque des revendications 7 à 11, l'injecteur de fluide (610, 10) comprenant une pompe (22), et l'injecteur de fluide (610, 10) en communication fluidique avec un ensemble de chemins de fluide (17, 1700) ;
l'au moins une chambre de fluide (612, 26, 28, 1716) comprenant en outre au moins une chambre de goutte-à-goutte (1716) en communication fluidique avec l'injecteur de fluide (610, 10) et au moins l'un d'un récipient de produit de contraste (26) et d'un récipient de solution saline (28), et
au moins un du ou des capteurs de rayonnement électromagnétique ou d'ultrasons (2000, 3000, 1484) étant positionné par rapport à l'au moins une chambre de goutte-à-goutte (1716) pour détecter la position du ménisque à l'interface liquide-gaz (801) du fluide contenu dans l'au moins une chambre de goutte-à-goutte (1716).

13. Procédé selon l'une quelconque des revendications 7 à 12, le dispositif d'affichage (118) comprenant une interface utilisateur graphique (5000), et la position du ménisque à l'interface liquide-gaz (801) du fluide dans l'au moins une chambre à fluide (612, 26, 28, 1716) étant affichée sur l'interface utilisateur graphique (5000).

14. Procédé selon l'une quelconque des revendications 7 à 13, l'injecteur de fluide (610, 10) comprenant un actionneur configuré pour actionner au moins un du ou des capteurs de rayonnement électromagnétique ou d'ultrasons (2000, 3000, 1484), et comprenant en outre l'étape de :
actionnement du ou des capteurs de rayonnement électromagnétique ou d'ultrasons (2000, 3000, 1484) pour qu'ils se déplacent en réponse à la position du ménisque à l'interface liquide-gaz (801) du fluide dans l'au moins une chambre de fluide (612, 26, 28, 1716).
